# EUROPÄISCHE PATENTANMELDUNG

(11) **EP 1 821 062 A2**
(43) Veröffentlichungstag der Anmeldung: **22.08.2007**
(21) Anmeldenummer: 07102171.1
(22) Anmeldetag: 12.02.2007
(51) Int. Cl.: G01B 3/30

(54) **Messlehre zur Spaltvermessung**

(30) Priorität: 15.02.2006 EP 06101721
(71) Anmelder: Zimmer GmbH, 8404 Winterthur (CH)
(72) Erfinder: Reinmuth, Jochen, 8406, Winterthur (CH); Filippi, Michael, 8200, Schaffhausen (CH)

(57) **Zusammenfassung**

Die vorliegende Erfindung betrifft eine Messlehre, umfassend eine Achse (11) und wenigstens ein Lehrelement (13, 15), das auf der Achse (11) befestigt ist, wobei das Lehrelement (13, 15) um die Achse (11) umlaufend eine diskrete Anzahl paarweise gegenüberliegend angeordneter Lehrflächen (29) umfasst, und wobei der Lehrflächenabstand (XS, S, M, Mo, L, XL) zwischen den paarweise gegenüberliegend angeordneten Lehrflächen (29) von Paar zu Paar unterschiedlich ist, um die Größe eines zwischen zwei Flächen (17, 19) gebildeten Spaltes, in den das Lehrelement (13, 15) im Gebrauch eingeführt ist, zu bestimmen oder einzustellen.

## Beschreibung

Die Erfindung betrifft allgemein die Bestimmung oder Einstellung der Größe eines Spalts, der zwischen zwei Flächen gebildet ist. Insbesondere betrifft die Erfindung die Bestimmung oder Einstellung der Spaltgröße an Instrumenten oder Implantaten, die bei der Durchführung von Operationen eingesetzt werden, insbesondere von Bandscheibenoperationen.

Operationsinstrumente sollen einfach handhabbar sein und - wenn sie zur Bestimmung oder Einstellung von Messgrößen dienen - eine gute Ablesbarkeit der jeweiligen Messgröße gewährleisten.

Diesen und anderen Anforderungen wird die Erfindung gerecht. Die hier angegebene Messlehre umfasst eine Achse und wenigstens ein Lehrelement, das auf der Achse befestigt ist. Das Lehrelement umfasst um die Achse umlaufend eine diskrete Anzahl paarweise gegenüberliegend angeordneter Lehrflächen, wobei der Lehrflächenabstand zwischen den paarweise gegenüberliegend angeordneten Lehrflächen von Paar zu Par unterschiedlich ist, um die Größe eines zwischen zwei Flächen gebildeten Spalts, in den das Lehrelement im Gebrauch eingeführt ist, zu bestimmen oder einzustellen.

Durch Drehen der Messlehre um die Achse werden also nacheinander Lehrflächenpaare wirksam, die sich hinsichtlich des Lehrflächenabstandes voneinander unterscheiden. Auf diese Weise kann der Benutzer durch Drehen der Messlehre einen vorhandenen Spalt ausmessen, um die Spaltgröße zu bestimmen, oder eine gewünschte Spaltgröße einstellen, indem die Messlehre in einer Drehstellung in den Spalt eingeführt wird, in welcher das den gewünschten Lehrflächenabstand aufweisende Lehrflächenpaar wirksam ist, woraufhin die den Spalt bildenden Flächen in Anlage mit den wirksamen Lehrflächen gebracht werden. Es ist auch möglich, mit Hilfe der Messlehre die beiden den Spalt bildenden Flächen voneinander weg zu bewegen, bis die gewünschte Spaltgröße eingestellt ist, indem die Messlehre zunächst in einer Drehstellung in den Spalt eingeführt wird, in welcher ein einen kleinen Lehrflächenabstand aufweisendes Lehrflächenpaar wirksam ist, woraufhin ausgehend von diesem Lehrflächenpaar durch Drehen der Messlehre ein Lehrflächenpaar mit dem gewünschten größeren Abstand in eine wirksame Stellung überführt wird, in welcher die den größeren Abstand aufweisenden Lehrflächen an den den Spalt bildenden Flächen anliegen.

Im Folgenden werden die unterschiedlichen Lehrflächenabstände, die von der Messlehre bereitgestellt werden, d.h. an deren Lehrelement zur Verfügung stehen, auch einfach als Größe der Messlehre bezeichnet.

Die hier angegebene Messlehre ist insbesondere in Verbindung mit Bandscheibenoperationen einsetzbar, bei denen ein Bandscheibenimplantat zwischen zwei benachbarte Wirbelkörper eingesetzt wird, das eine kraniale Implantatplatte und eine kaudale Implantatplatte sowie einen Einsatz umfasst, der sich im implantierten Zustand zwischen den beiden Implantatplatten befindet. Ein derartiges Bandscheibenimplantat ist unter dem Namen "Dynardi" der Anmelderin bekannt. Ein Operationsschritt zum Einsetzen dieses Bandscheibenimplantats beinhaltet eine Distraktion der beiden Implantatplatten, wenn diese bereits in den Zwischenraum zwischen den beiden benachbarten Wirbelkörpern eingesetzt sind, um bei Erreichen des gewünschten Abstandes zwischen den beiden distrahierten Implantatplatten den Einsatz zwischen die beiden Implantatplatten einbringen zu können. Die Distraktion der beiden Implantatplatten erfolgt mit Hilfe eines Distraktionsinstruments, das an den distalen Enden zweier Arme jeweils einen Plattenhalter aufweist, an dem eine Implantatplatte lösbar befestigt ist. Der Abstand zwischen den beiden Implantatplatten ist dabei durch den Abstand zwischen den beiden Plattenhaltern festgelegt, wobei zwischen den beiden Plattenhaltern ein von zwei Flächen gebildeter Spalt vorhanden ist. In den bei der Bestimmung oder Einstellung des Abstandes zwischen den Implantatplatten auftretenden Stellungen der Arme des Distraktionsinstruments verlaufen die beiden den Spalt zwischen den Plattenhaltern bildenden Flächen der Plattenhalter parallel zueinander.

Mit der hier angegebenen Messlehre kann der Operateur sicherstellen, dass zwischen den beiden Plattenhaltern und damit zwischen den beiden Implantatplatten die gewünschte Spaltgröße eingestellt ist, bevor der Einsatz zwischen die Implantatplatten eingeführt wird. Die mit der Messlehre einstellbaren oder bestimmbaren Größen, d.h. die am Lehrelement ausgebildeten Lehrflächenabstände, entsprechen dabei im Sinne einer eindeutigen Zuordnung den dem Operateur zur Verfügung stehenden Implantatgrößen bzw. Größen des zwischen die Implantatplatten einzubringenden Einsatzes.

Auf weitere Einzelheiten dieses Bandscheibenimplantats sowie des hierfür vorgesehenen Operationsverfahrens braucht an dieser Stelle nicht näher eingegangen zu werden.

Die paarweise gegenüberliegend angeordneten Lehrflächen der hier angegebenen Messlehre verlaufen insbesondere parallel zueinander. Zwingend erforderlich ist dies aber nicht. In Abhängigkeit von der relativen Orientierung der beiden den Spalt bildenden Flächen können die ein Lehrflächenpaar bildenden, gegenüberliegend angeordneten Lehrflächen auch gegeneinander geneigt angeordnet sein. Dabei ist es grundsätzlich möglich, dass eine Messlehre sowohl ein oder mehrere Lehrflächenpaare mit parallel zueinander verlaufenden Lehrflächen als auch ein oder mehrere Lehrflächenpaare mit geneigt zueinander verlaufenden Lehrflächen umfasst.

In einem Ausführungsbeispiel ist vorgesehen, dass alle an dem Lehrelement ausgebildeten Lehrflächenpaare jeweils von Lehrflächen gebildet sind, die parallel zueinander verlaufen.

Die Anwendung der hier angegebenen Messlehre ist nicht auf Bandscheibenoperationen beschränkt. Vielmehr ist die Messlehre grundsätzlich in allen Operationsverfahren einsetzbar, in deren Verlauf die Größe eines zwischen zwei Flächen gebildeten Spalts oder Zwischenraums bestimmt oder eingestellt werden soll. Insbesondere ist die angegebene Messlehre in Verbindung mit Distraktionsinstrumenten einsetzbar, wie sie bei verschiedenen Operationen zum Einsatz kommen, beispielsweise auch beim Einsetzen von Knieimplantaten.

Die hier angegebene Messlehre beruht auf einem einfachen Messprinzip und ist daher für den Operateur leicht zu handhaben. Die um die Achse umlaufend angeordneten Lehrflächenpaare ermöglichen eine einfache und eindeutige Positionierung der mit dem Lehrelement bereitgestellten Größen bezüglich der den Spalt bildenden Flächen.

In einer Ausführungsform sind für jedes Paar die beiden gegenüberliegend angeordneten Lehrflächen achssymmetrisch angeordnet.

In einer weiteren Ausgestaltung ist vorgesehen, dass in einer Umlaufrichtung der Lehrflächenabstand ausgehend von einem Paar mit einem kleinsten Lehrflächenabstand bis zu einem Paar mit einem größten Lehrflächenabstand von Paar zu Paar ansteigt.

Gemäß einem weiteren Ausführungsbeispiel beträgt ausgehend von einem Paar mit einem kleinsten Lehrflächenabstand A der Lehrflächenabstand des n-ten sich in Umlaufrichtung anschließenden Paares A+n*x, wobei x für das Lehrelement eine Konstante ist. Jeweils zwei bei Drehung des Lehrelements um die Achse aufeinander folgende Lehrflächenabstände (Größen) unterscheiden sich somit stets um den gleichen Betrag.

In einer weiteren Ausgestaltung der hier angegebenen Messlehre ist der Winkel zwischen jeweils zwei in Umlaufrichtung aufeinander folgenden Lehrflächen jeweils gleich. Insbesondere ist der Winkel kleiner oder gleich 60°, wobei insbesondere der Winkel 45° beträgt.

In einem weiteren Ausführungsbeispiel ist vorgesehen, dass die Lehrflächen von in Umlaufrichtung unmittelbar aufeinander folgenden Paaren unmittelbar aneinander angrenzen, insbesondere unter Ausbildung von Übergangsbereichen.

Die gegebenenfalls vorgesehenen Übergangsbereiche sind insbesondere abgerundet. Durch eine entsprechende Ausgestaltung, insbesondere Abrundung, der Übergangsbereiche kann erreicht werden, dass aufgrund der Übergänge sich ergebende vorübergehende Spaltvergrößerungen, die über den größeren der beiden bei einem Größenwechsel beteiligten Lehrflächenabstände hinausgehen, möglichst klein gehalten werden. Bei Anwendung der hier angegebenen Messlehre bei Operationen in Verbindung mit Distraktionsinstrumenten können bei einem Wechsel zwischen zwei Größen auftretende Über-Distraktionen also in einem akzeptierbaren Rahmen gehalten werden.

In einem weiteren Ausführungsbeispiel sind auf der Achse wenigstens ein erstes Lehrelement und ein zweites Lehrelement in axialer Richtung hintereinander angeordnet, wobei die beiden Lehrelemente hinsichtlich der Abstände ihrer Lehrflächen unterschiedlich ausgebildet sind. Hierdurch lässt sich eine mehrstufige Messlehre realisieren, die es dem Benutzer ermöglicht, zwischen unterschiedlichen Gruppen oder Sätzen von aufeinander folgenden Lehrflächenabständen, die jeweils an einem Lehrelement ausgebildet sind, je nach Bedarf zu wechseln. Dieser Wechsel kann einfach dadurch bewerkstelligt werden, dass die Messlehre entlang der Achse weiter in den Spalt eingeführt oder aus dem Spalt herausgezogen wird, um das jeweils gewünschte Lehrelement mit den den Spalt bildenden Flächen zusammenwirken zu lassen.

Insbesondere sind das erste Lehrelement und das zweite Lehrelement unmittelbar aneinander angrenzend angeordnet. Das erste Lehrelement und das zweite Lehrelement sind insbesondere einstückig miteinander ausgebildet.

In einer weiteren Ausgestaltung der hier angegebenen Messlehre stimmen das erste Lehrelement und das zweite Lehrelement hinsichtlich wenigstens eines Lehrflächenabstandes überein. Hierdurch wird erreicht, dass mit einem Wechsel zwischen den beiden Lehrelementen nicht zwangsläufig eine Änderung der Größe verbunden ist.

Insbesondere ist der größte Lehrflächenabstand des ersten Lehrelements gleich dem kleinsten Lehrflächenabstand des zweiten Lehrelementes. Hierdurch schließen die von beiden Lehrelementen jeweils zur Verfügung gestellten Größen unter Ausbildung eines Lehrflächenabstandes gleicher Größe aneinander an, so dass die beiden Lehrelemente gemeinsam eine - je nach Umlaufrichtung aufsteigende oder absteigende - Folge von Größen bilden, wobei eine Größe an beiden Lehrelementen zur Verfügung steht.

Eine Übereinstimmung hinsichtlich eines oder mehrerer Lehrflächenabstände zwischen den beiden Lehrelementen ist nicht zwingend, d.h. die von den beiden Lehrelementen insgesamt gebildete, je nach Drehrichtung aufsteigende oder absteigende Folge von Größen kann auch so ausgestaltet sein, dass jede Größe nur ein einziges Mal innerhalb der Folge existiert. Ebenso ist in einer möglichen Ausführungsform vorgesehen, dass die beiden Lehrelemente hinsichtlich mehrerer Lehrflächenabstände übereinstimmen.

In einer weiteren Ausgestaltung der hier angegebenen Messlehre sind ein Lehrflächenpaar des ersten Lehrelements und ein Lehrflächenpaar des zweiten Lehrelements, die den gleichen Lehrflächenabstand aufweisen, an der gleichen Achsumlaufposition angeordnet. Ein Wechsel zwischen den beiden Lehrelementen ist hierdurch weder mit einer Änderung des Lehrflächenabstandes noch mit einer Drehung der Messlehre um die Achse verbunden. Vielmehr braucht der Benutzer die Messlehre lediglich in axialer Richtung weiter in den Spalt einzuführen oder aus dem Spalt herausziehen, um von dem einen Lehrelement auf das andere Lehrelement zu wechseln.

Gemäß einer weiteren Ausführungsform ist vorgesehen, dass ausgehend von einem am ersten Lehrelement ausgebildeten Paar mit einem kleinsten Lehrflächenabstand A bis zu einem am zweiten Lehrelement ausgebildeten Paar mit dem größten Lehrflächenabstand der Lehrflächenabstand des n-ten sich in Umlaufrichtung anschließenden Paares A+n*x beträgt, wobei x eine Konstante ist.

Alternativ zu Ausführungsformen mit einem einzigen oder mit zwei Lehrelementen können Ausführungsformen der hier angegebenen Messlehre vorgesehen sein, bei denen mehr als zwei Lehrelemente vorhanden und in axialer Richtung hintereinander angeordnet sind. In einer möglichen Ausgestaltung einer solchen Messlehre kann vorgesehen sein, dass jeweils zwei in axialer Richtung unmittelbar aufeinander folgende Lehrelemente so ausgebildet sind, wie es vorstehend in Verbindung mit Ausführungsbeispielen, bei denen zwei Lehrelemente vorgesehen sind, erläutert wurde.

In einer Ausführungsform umfasst die Achse eine Messseite und eine Manipulationsseite, wobei das Lehrelement mit dem kleinsten Lehrflächenabstand am weitesten messseitig und das Lehrelement mit dem größten Lehrflächenabstand am weitesten manipulationsseitig angeordnet ist.

Gemäß einer weiteren Ausgestaltung der hier angegebenen Messlehre ist diese mit einem Griff versehen, der auf der Achse in axialem Abstand von dem Lehrelement befestigt und an dem bezüglich der Umlaufrichtung die Stellung des Lehrelements in dem Spalt ablesbar ist. Hierdurch kann der Benutzer anhand des für ihn besser einsehbaren Griffs die momentane Stellung des Lehrelements in dem Spalt bestimmen oder verifizieren.

Dabei ist insbesondere zumindest ein Teil des Griffs hinsichtlich der äußeren Form dem Lehrelement nachgebildet. Hierdurch wird eine besonders intuitive Benutzung der hier angegebenen Messlehre ermöglicht.

In einem Ausführungsbeispiel ist vorgesehen, dass das Lehrelement und der Griff in Umlaufrichtung zueinander ausgerichtet sind. Die Bestimmung der momentanen Stellung des Lehrelements in dem Spalt wird hierdurch besonders einfach.

Wie eingangs bereits angedeutet, ist in einem Ausführungsbeispiel die Messlehre als Instrument zur intraoperativen Bestimmung der Größe eines Spalts ausgebildet, der zwischen Implantatplatten eines Bandscheibenimplantats oder zwischen Halteinstrumenten zum Halten von Implantatplatten eines Bandscheibenimplantats gebildet ist.

Alle sowohl in den Ansprüchen als auch in der Beschreibung und in den Zeichnungen in Verbindung mit den hier angegebenen Gegenständen gegebenenfalls verwendeten und gemäß der fachüblichen Konvention gewählten Ausrichtungs-, Positionierungs-, Orientierungs- und Richtungsangaben, die insbesondere anatomische Achsen, Ebenen, Richtungen im Raum und Bewegungsrichtungen betreffen, sind dem Fachmann bekannt und beziehen sich gegebenenfalls auf den implantierten Zustand.

Nachfolgend wird die Erfindung anhand von in der Zeichnung illustrierten Ausführungsbeispielen näher erläutert. Dabei sollen die Ausführungsbeispiele und die Zeichnungen nur instruktiv verstanden werden und nicht zur Einschränkung der in den Ansprüchen beschriebenen Gegenstände dienen. Die Darstellungen in der Zeichnung sind vereinfacht; für das Verständnis der Erfindung nicht notwendige Einzelheiten sind weggelassen worden.

In der Zeichnung zeigt:
- Fig. 1: eine Ausführungsform einer Messlehre der hier angegebenen Art in zwei unterschiedlichen Drehstellungen,
- Fig. 2: eine Ausführungsform eines Lehrelements für eine Messlehre der hier angegebenen Art,
- Fig. 3: eine schematisierte Ansicht auf die Stirnseite eines Lehrelements der hier angegebenen Art zur Erläuterung des Funktionsprinzips,
- Fig. 4: eine Ansicht auf die Stirnseite einer Ausführungsform eines Lehrelements der hier angegebenen Art,
- Fig. 5: eine Ausführungsform eines Griffs einer Messlehre der hier angegebenen Art in zwei unterschiedlichen Drehstellungen, und
- Fig. 6: eine Darstellung zur Erläuterung einer möglichen Anwendung einer Messlehre der hier angegebenen Art.

Die Ausführungsbeispiele dienen dem besseren Verständnis der Erfindung und sollen nicht zu einer Einschränkung der in den Ansprüchen angegebenen Erfindung herangezogen werden.

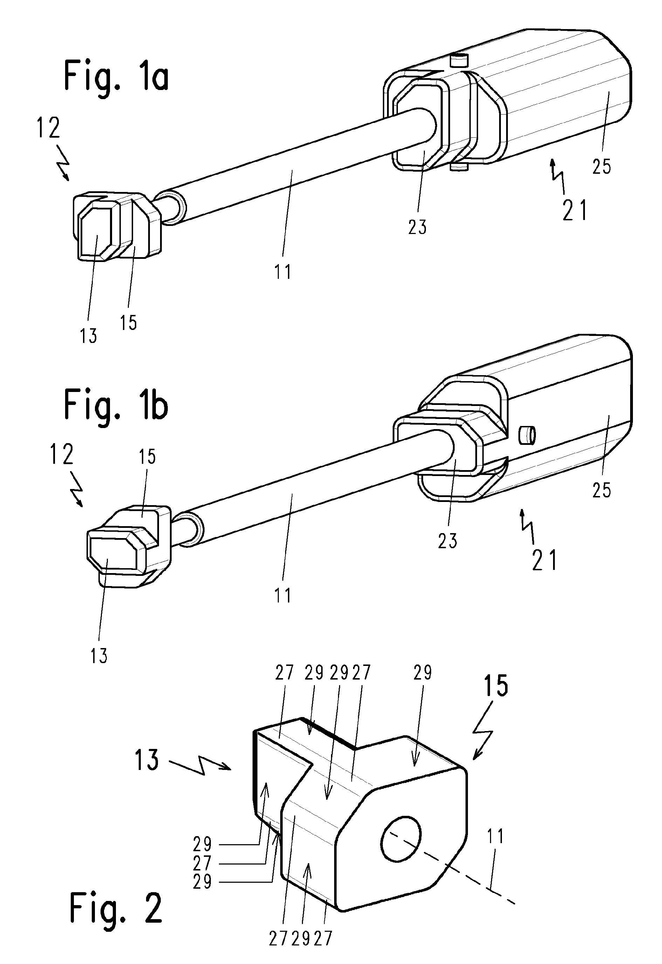

Die in Fig. 1 dargestellte Messlehre umfasst eine Achse 11, die hier in Form eines Stabs mit kreiszylindrischem Querschnitt ausgebildet ist.

Die Achse 11 umfasst eine Messseite und eine Manipulationsseite. Messseitig ist eine Lehre 12 an der Achse 11 befestigt, die ein erstes Lehrelement 13 und ein zweites Lehrelement 15 umfasst, die in axialer Richtung unmittelbar aneinander angrenzend hintereinander angeordnet und einstückig miteinander ausgebildet sind. Auf den Aufbau der Lehrelemente 13, 15 wird nachstehend näher eingegangen.

Am manipulationsseitigen Ende der Achse 11 ist ein Griff 21 befestigt, über welchen die hier angegebene Messlehre von einem Benutzer gehandhabt werden kann. Der Griff 21 umfasst ein erstes Griffelement 23 und ein zweites Griffelement 25, die entsprechend dem Aufbau der Lehre 12 in axialer Richtung unmittelbar aneinander angrenzend hintereinander angeordnet und einstückig miteinander ausgebildet sind.

Der Griff 21 ist hinsichtlich der äußeren Form der Lehre 12 nachgebildet, wobei das erste Griffelement 23 dem ersten Lehrelement 13 und das zweite Griffelement 25 dem zweiten Lehrelement 15 nachgebildet ist.

Die perspektivische Ansicht der Fig. 2 zeigt eine Ausführungsform einer wiederum ein erstes Lehrelement 13 und ein zweites Lehrelement 15 umfassenden Lehre, wobei eine manipulationsseitige Stirnseite des zweiten Lehrelements 15 erkennbar ist. Die Achse 11, an welcher die Lehre befestigt ist, ist in Fig. 2 lediglich durch eine gestrichelte Linie angedeutet.

Jedes Lehrelement 13, 15 umfasst eine Mehrzahl von - hier drei - Lehrflächenpaaren, die jeweils zwei einander gegenüberliegende, parallel zueinander verlaufende Lehrflächen 29 umfassen. Für jedes Paar sind die beiden gegenüberliegend angeordneten Lehrflächen 29 bezüglich der Achse 11 symmetrisch derart angeordnet, dass eine senkrecht zu den Lehrflächen 29 verlaufende Gerade auch senkrecht zur Achse 11 verläuft, wobei der Abstand zur Achse 11 für beide Lehrflächen 29 der gleiche ist.

Für jedes Lehrelement 13, 15 grenzen die Lehrflächen 29 von in Achsumlaufrichtung unmittelbar aufeinander folgenden Lehrflächenpaaren unter Ausbildung von Übergangsbereichen 27 aneinander an. Die Übergangsbereiche 29 sind abgerundet.

Des Weiteren ist für jedes Lehrelement 13, 15 der Lehrflächenabstand zwischen den paarweise gegenüberliegend angeordneten Lehrflächen 29 von Paar zu Paar unterschiedlich. Hierdurch weist jedes Lehrelement 13, 15 drei Größen auf, wobei hier der Einfachheit halber mit "Größe" bestimmter Lehrflächenabstand, also ein bestimmtes Lehrflächenpaar, bezeichnet wird.

Fig. 2 ist zu entnehmen, dass die beiden Lehrelemente 13, 15 hinsichtlich einer Größe übereinstimmen, wobei die betreffenden Lehrflächenpaare an der gleichen Achsumlaufposition angeordnet sind. Die betreffenden Lehrflächen 29 gehen somit in axialer Richtung betrachtet unter Ausbildung einer einzigen größeren durchgehenden Gesamtfläche ineinander über.

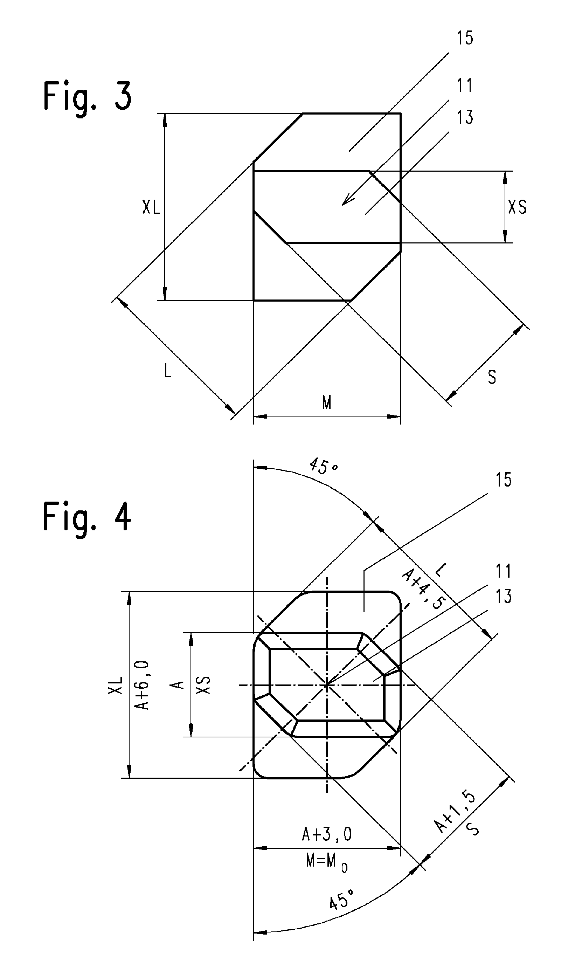

Auf die Geometrie der beiden Lehrelemente 13, 15 wird nachstehend anhand der Fig. 3 und 4 näher eingegangen.

In Fig. 3 ist in einer schematisierten Stirnansicht auf das am weitesten messseitig angeordnet Lehrelement 13 der Lehre das dem Aufbau der Lehrelemente 13, 15 zugrunde liegende Konzept veranschaulicht. Die Übergänge zwischen in Achsumlaufrichtung unmittelbar aufeinander folgenden Lehrflächen sind hier der Einfachheit halber nicht abgerundet dargestellt.

Die von den beiden Lehrelementen 13, 15 insgesamt zur Verfügung gestellten fünf Größen sind hier mit XS, S, M = M₀, L und XL bezeichnet. Dabei stehen an dem messseitigen Lehrelement 13 die Größen XS, S und M zur Verfügung, während das manipulationsseitige Lehrelement 15 die Größen M₀, L und XL bereitstellt. Wie bereits erwähnt und in Fig. 3 angegeben, gilt M = M₀, d.h. eine Größe - hier die mittlere Größe - ist an beiden Lehrelementen 13, 15 vorgesehen, und zwar an der gleichen Achsumlaufposition.

Der Benutzer kann somit unter Verwendung zunächst des messseitigen Lehrelements 13 ausgehend von der kleinsten Größe XS, also dem kleinsten insgesamt zur Verfügung stehenden Lehrflächenabstand, jeweils durch Drehen der Messlehre um die Achse 11 von Lehrflächenpaar zu Lehrflächenpaar und somit jeweils zur nächst höheren Größe wechseln, wobei zum Einstellen der Größe L zunächst bei eingestellter Größe M = M₀ eine axiale Verschiebung erfolgt, um von dem ersten Lehrelement 13 (Größe M) zu dem zweiten Lehrelement 15 (Größe M₀) zu wechseln, um dann durch Weiterdrehen der Messlehre als nächstes die Größe L und dann die Größe XL zu erreichen.

Die jeweils eingestellte bzw. wirksame Größe ist dadurch festgelegt, dass die Lehrflächen, welche durch ihren Abstand die jeweilige Größe bestimmen, bezüglich Flächen ausgerichtet sind, die einen Spalt oder einen sonstigen Zwischenraum bilden oder begrenzen, dessen Größe oder Höhe bestimmt oder eingestellt werden soll. In diesen Spalt oder Zwischenraum wird die Lehre derart eingeführt, dass das jeweilige Lehrelement 13, 15 über seine Lehrflächen mit den den Spalt bzw. Zwischenraum bildenden Flächen zusammenwirken kann.

Fig. 4 ist eine Ansicht entsprechend Fig. 3, wobei Fig. 4 die Systematik zu entnehmen ist, die der von den beiden Lehrelementen 13, 15 bereitgestellten Folge von einstellbaren Größen zugrunde liegt.

Wenn der kleinste insgesamt vorhandene, am ersten Lehrelement 13 ausgebildete Lehrflächenabstand, also die Größe XS, mit A bezeichnet wird, dann vergrößert sich der Lehrflächenabstand jeweils zur nächst höheren Größe um einen für die gesamte Messlehre einheitlichen Betrag x, wobei x in dem hier dargestellten Ausführungsbeispiel 1,5 mm beträgt. Da die Größe M = Mo an beiden Lehrelementen 13, 15 vorgesehen ist, sind insgesamt fünf Größen vorhanden, so dass für die Grö-ße XL ein Lehrflächenabstand von A + 6,0 mm gegeben ist.

Fig. 4 ist des Weiteren zu entnehmen, dass für jedes Lehrelement 13, 15 jeweils zwei in Achsumlaufrichtung unmittelbar aufeinander folgende Lehrflächen den gleichen Winkel einschließen, der in diesem Ausführungsbeispiel 45° beträgt. Zum Wechseln von einer Größe zur nächst höheren oder nächst niedrigeren Größe ist die Messlehre folglich von dem Benutzer stets um den gleichen Winkelbetrag zu drehen.

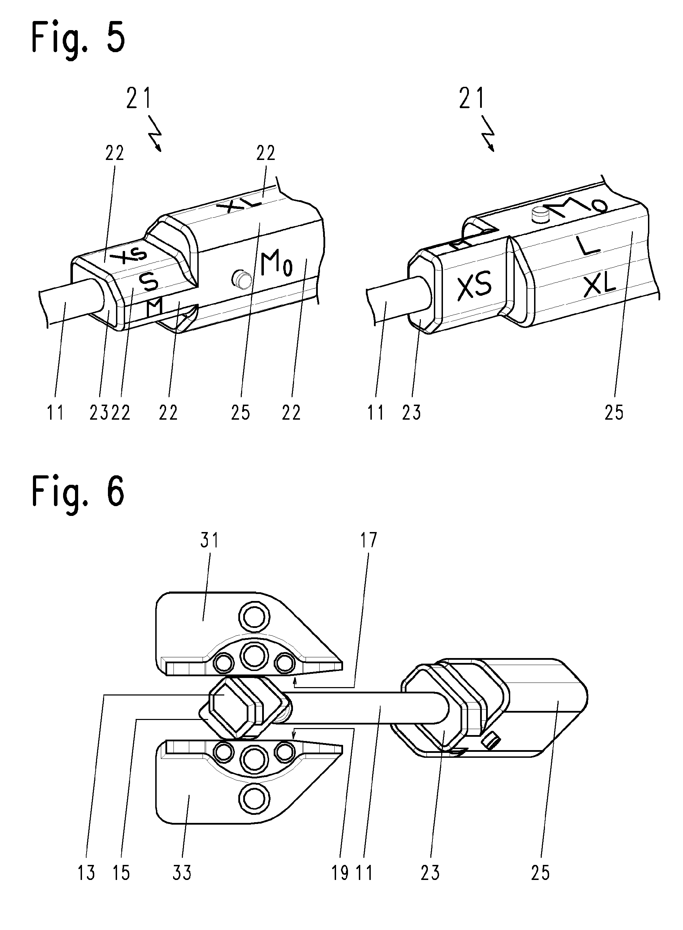

Wie vorstehend in Verbindung mit Fig. 1 bereits erwähnt, ist der in Fig. 5 dargestellte Griff 21 der Messlehre der messseitig angeordneten Lehre nachgebildet. Hinsichtlich der Abstände der den Lehrflächen 29 entsprechenden Griffflächen 22 sind das erste Griffelement 23 und das erste Lehrelement 13 einerseits und das zweite Griffelement 25 und das zweite Lehrelement 15 andererseits identisch ausgeführt. Zusätzlich sind die Griffflächen 22 jeweils mit einer die entsprechende Größe XS, S, M, M₀, L oder XL bezeichnenden Beschriftung versehen.

Fig. 6 zeigt eine mögliche Anwendung der hier angegebenen Messlehre. Die Messlehre ist hier als ein Instrument zur intraoperativen Bestimmung der Größe eines Spalts ausgebildet, der von einer kranialen Fläche 17 eines kranialen Plattenhalters 31 und von einer kaudalen Fläche 19 eines kaudalen Plattenhalters 33 gebildet wird.

Zum Bestimmen oder Einstellen des Plattenabstands kann die Messlehre zunächst so weit zwischen die beiden Plattenhalter 31, 33 eingeführt werden, dass nur das am weitesten messseitig gelegene erste Lehrelement 13 wirksam ist, an welchem das Lehrflächenpaar mit der kleinsten Größe XS ausgebildet ist. Beim Einführen des ersten Lehrelements 13 sind dabei die die Größe XS festlegenden, einander gegenüberliegenden Lehrflächen mit den spaltbildenden Flächen 17, 19 der Plattenhalter 31, 33 ausgerichtet.

Anschließend wird die Messlehre um die Achse 11 derart gedreht, dass als nächstes die Größe S wirksam wird. Ausgehend von der Größe S wird durch erneutes Drehen der Messlehre um die Achse 11 um den gleichen Winkelbetrag wie zuvor die Größe M eingestellt. Fig. 6 zeigt die Messlehre in einer Zwischenstellung zwischen den beiden Größen S und M, in welcher die entsprechenden Übergangsbereiche 27 (Fig. 2) mit den spaltbildenden Flächen 17, 19 zusammenwirken. Die Abrundung der Übergangsbereiche 27 reduziert das bei einem Größenwechsel mögliche Abstandsübermaß zwischen den beiden Plattenhaltern 31, 33, das über den größeren der beiden bei dem Wechsel beteiligten Abstände hinausgeht.

Um von der Größe M = M₀ auf die Größe L zu wechseln, die am zweiten, manipulationsseitigen Lehrelement 15 ausgebildet ist, wird die Messlehre weiter in den Spalt hineingeschoben, bis die beiden die Größe M₀ bildenden Lehrflächen des zweiten Lehrelements 15 wirksam sind. Ausgehend von dieser Größe können dann mittels des zweiten Lehrelements 15 die beiden Größen L und XL nacheinander eingestellt werden.

Analog kann gegebenenfalls eine Reduzierung der Spaltgröße in umgekehrter Dreh- bzw. Verschieberichtung erfolgen.

Im Lichte der hier gemachten Ausführungen eröffnen sich dem Fachmann weitere Ausführungsformen der in den Ansprüchen angegebenen Gegenstände, welche hier nicht abschließend dargestellt werden können.

### Bezugszeichenliste

- 11: Achse
- 12: Lehre
- 13: erstes Lehrelement
- 15: zweites Lehrelement
- 17: kraniale spaltbildende Fläche
- 19: kaudale spaltbildende Fläche
- 21: Griff
- 22: Grifffläche
- 23: erstes Griffelement
- 25: zweites Griffelement
- 27: Übergangsbereich
- 29: Lehrfläche
- 31: kraniales Halteinstrument, Plattenhalter
- 33: kaudales Halteinstrument, Plattenhalter

- A: kleinster Lehrflächenabstand
- XS: Lehrflächenabstand, Größe
- X: Lehrflächenabstand, Größe
- M: Lehrflächenabstand, Größe
- M₀: Lehrflächenabstand, Größe
- L: Lehrflächenabstand, Größe
- XL: Lehrflächenabstand, Größe

## Patentansprüche

1. Messlehre, umfassend
eine Achse (11) und
wenigstens ein Lehrelement (13, 15), das auf der Achse (11) befestigt ist,
wobei das Lehrelement (13, 15) um die Achse (11) umlaufend eine diskrete Anzahl paarweise gegenüberliegend und insbesondere achssymmetrisch angeordneter Lehrflächen (29) umfasst, und
wobei der Lehrflächenabstand (XS, S, M, Mo, L, XL) zwischen den paarweise gegenüberliegend angeordneten Lehrflächen (29) von Paar zu Paar unterschiedlich ist, um die Größe eines zwischen zwei Flächen (17, 19) gebildeten Spaltes, in den das Lehrelement (13, 15) im Gebrauch eingeführt ist, zu bestimmen oder einzustellen.

2. Messlehre nach Anspruch 1,
**dadurch gekennzeichnet,**
**dass** in einer Umlaufrichtung der Lehrflächenabstand (XS, S, M, Mo, L, XL) ausgehend von einem Paar mit einem kleinsten Lehrflächenabstand (XS) bis zu einem Paar mit einem größten Lehrflächenabstand (XL) von Paar zu Paar ansteigt.

3. Messlehre nach einem der vorhergehenden Ansprüche,
**dadurch gekennzeichnet,**
**dass** ausgehend von einem Paar mit einem kleinsten Lehrflächenabstand A der Lehrflächenabstand des n-ten sich in Umlaufrichtung anschließenden Paares A+n*x beträgt, wobei x für das Lehrelement (13, 15) eine Konstante ist.

4. Messlehre nach einem der vorhergehenden Ansprüche,
**dadurch gekennzeichnet,**
**dass** der Winkel zwischen jeweils zwei in Umlaufrichtung aufeinander folgenden Lehrflächen (29) jeweils gleich ist, und wobei der Winkel insbesondere kleiner oder gleich 60° ist und weiterhin insbesondere 45° beträgt.

5. Messlehre nach einem der vorhergehenden Ansprüche,
**dadurch gekennzeichnet,**
**dass** auf der Achse (11) wenigstens ein erstes Lehrelement (13) und ein zweites Lehrelement (15) in axialer Richtung hintereinander angeordnet sind, wobei die beiden Lehrelemente (13, 15) hinsichtlich der Abstände ihrer Lehrflächen (29) unterschiedlich ausgebildet sind.

6. Messlehre nach Anspruch 5,
**dadurch gekennzeichnet,**
**dass** das erste Lehrelement (13) und das zweite Lehrelement (15) unmittelbar aneinander grenzend angeordnet sind und insbesondere das erste Lehrelement (13) und das zweite Lehrelement (15) einstückig miteinander ausgebildet sind.

7. Messlehre nach einem der Ansprüche 5 oder 6,
**dadurch gekennzeichnet,**
**dass** der größte Lehrflächenabstand (M) des ersten Lehrelementes (13) gleich dem kleinsten Lehrflächenabstand (Mo) des zweiten Lehrelementes (15) ist, wobei insbesondere ein Lehrflächenpaar des ersten Lehrelementes (13) und ein Lehrflächenpaar des zweiten Lehrelementes (15), die den gleichen Lehrflächenabstand (M; Mo) aufweisen, an der gleichen Achsumlaufposition angeordnet sind.

8. Messlehre nach einem der vorhergehenden Ansprüche,
**dadurch gekennzeichnet,**
**dass** eine Mehrzahl von Lehrelementen (13, 15) vorgesehen ist, wobei jeweils zwei in axialer Richtung unmittelbar aufeinander folgende Lehrelemente nach einem der Ansprüche 5 bis 7 ausgebildet sind.

9. Messlehre nach Anspruch 8,
**dadurch gekennzeichnet,**
**dass** die Achse (11) eine Messseite und eine Manipulationsseite umfasst, wobei das Lehrelement (13) mit dem kleinsten Lehrflächenabstand (XS) am weitesten messseitig und das Lehrelement (15) mit dem größten Lehrflächenabstand (XL) am weitesten manipulationsseitig angeordnet ist.

10. Messlehre nach einem der vorhergehenden Ansprüche,
**dadurch gekennzeichnet,**
**dass** auf der Achse (11) in axialem Abstand von dem Lehrelement (13, 15) ein Griff (21) befestigt ist, an dem bezüglich der Umlaufrichtung die Stellung des Lehrelements (13, 15) in dem Spalt ablesbar ist.
